# EUROPEAN PATENT APPLICATION

(11) **EP 1 352 911 A1**
(43) Date of publication of application: **15.10.2003**
(21) Application number: 02729576.5
(22) Date of filing: 15.01.2002
(51) Int. Cl.: C07K 7/14, A23J 3/16, A23J 3/34

(54) **ANGIOTENSIN CONVERTING ENZYME INHIBITORS**

(30) Priority: 16.01.2001 JP 2001007400; 04.10.2001 JP 2001308974
(71) Applicant: Ajinomoto Co., Inc., Tokyo 104-0031 (JP)
(72) Inventor: KODERA, Tomohiro, c/o Central Research Lab., Kawasakii-Shi, Kanagawa 210-0801 (JP); NIO, Noriki, c/o Central Research Lab., Kawasaki-Shi, Kanagawa 210-0801 (JP)
(74) Representative: Strehl Schübel-Hopf & Partner
(86) International application number: JP0200194
(87) International publication number: WO02055546

(57) **Abstract**

the object of the present invention is to provide a peptide having angiotensin converting enzyme inhibitory activity also improved in the taste, and to provide a method of producing an angiotensin converting enzyme inhibitors comprising at least one of such enzymes. Another object of the present invention is to provide an antihypertensive comprising a peptide having angiotensin converting enzyme inhibitory activity. Thus, the present invention is any of the five peptides represented by the following formula (1) to (5) and the salts thereof, soybean protein hydrolysates containing these peptides and the method of producing these peptides or the salts thereof comprising hydrolyzing soybean proteins with protease D3:(1)Tyr-Val-Val-Phe-Lys; (2)Pro-Asn-Asn-Lys-Pro-Phe-Gln; (3)Asn-Trp-Gly-Pro-Leu-Val; (4)lle-Pro-Pro-Gly-Val-Pro-Tyr-Trp-Thr; (5)Thr-Pro-Arg-Val-Phe.

SEQUENCE LISTING

<110> Ajinomoto Co., Inc.

<120> Angiotensin converting enzyme inhibitor and a method of
producing the inhibitor

<130> EPA-63210

<140>
<141>

<150> JP 2001-7400
<151> 2001-01-16

<150> JP 2001-308974
<151> 2001-10-04

<160> 14

<170> PatentIn Ver. 2.1

<210> 1
<211> 5
<212> PRT
<213> Glycine max

<400> 1
Tyr Val Val Phe Lys

1                    5

<210> 2
<211> 7
<212> PRT
<213> Glycine max

<400> 2
Pro Asn Asn Lys Pro Phe Gln
1                    5

<210> 3
<211> 6
<212> PRT
<213> Glycine max

<400> 3
Asn Trp Gly Pro Leu Val
1                    5

<210> 4
<211> 9
<212> PRT
<213> Glycine max

<400> 4

Ile Pro Pro Gly Val Pro Tyr Trp Thr
1               5

<210> 5
<211> 5
<212> PRT
<213> Glycine max

<400> 5
Thr Pro Arg Val Phe
1               5

<210> 6
<211> 1392
<212> DNA
<213> Glycine max

<220>
<221> mat_peptide
<222> (397)..(1392)

<220>
<221> CDS
<222> (1)..(1392)

<400> 6
atg acc atg gct gcg atc gtg ctc ctg ttc acg gtc ttt gcc gtt tcc     48

Met Thr Met Ala Ala Ile Val Leu Leu Phe Thr Val Phe Ala Val Ser
-130 -125 -120

tcc gcc cta gac atg tcg ata atc tcg tac gac agc gcc cac gcg gac 96
Ser Ala Leu Asp Met Ser Ile Ile Ser Tyr Asp Ser Ala His Ala Asp
-115 -110 -105

aag gcc gcc acg ttg cgc acc gag gag gag ctg atg tcc atg tac gag 144
Lys Ala Ala Thr Leu Arg Thr Glu Glu Glu Leu Met Ser Met Tyr Glu
-100 -95 -90 -85

cag tgg ctc gtg aag cac ggg aag gtg tac aac gcg ctc ggc gag aag 192
Gln Trp Leu Val Lys His Gly Lys Val Tyr Asn Ala Leu Gly Glu Lys
-80 -75 -70

gag aag cgc ttc cag atc ttc aag gac aac ctg cga ttc atc gac gac 240
Glu Lys Arg Phe Gln Ile Phe Lys Asp Asn Leu Arg Phe Ile Asp Asp
-65 -60 -55

cac aac tcc gcg gag gac cga acc tac aag ctc gga ctg aac cgg ttc 288
His Asn Ser Ala Glu Asp Arg Thr Tyr Lys Leu Gly Leu Asn Arg Phe
-50 -45 -40

gct gat ctc acc aac gag gaa tac agg gcc aag tac ttg gga acc aag 336
Ala Asp Leu Thr Asn Glu Glu Tyr Arg Ala Lys Tyr Leu Gly Thr Lys
-35 -30 -25

atc gat ccc aac cgg agg ctc gga aag acc ccg agc aac cgc tac gcg 384
Ile Asp Pro Asn Arg Arg Leu Gly Lys Thr Pro Ser Asn Arg Tyr Ala

-20                    -15                    -10                    -5

cca cgt gtc ggc gac aaa ttg cct gat tcc gtt gat tgg agg aag gaa    432
Pro Arg Val Gly Asp Lys Leu Pro Asp Ser Val Asp Trp Arg Lys Glu
                -1   1                   5                   10

ggt gct gtt cct cct gtc aaa gac caa gga ggc tgt ggg agc tgt tgg    480
Gly Ala Val Pro Pro Val Lys Asp Gln Gly Gly Cys Gly Ser Cys Trp
            15                  20                  25

gca ttc tca gca atc ggt gca gta gaa gga ata aat aag ata gta aca    528
Ala Phe Ser Ala Ile Gly Ala Val Glu Gly Ile Asn Lys Ile Val Thr
        30                  35                  40

ggc gaa ctg att tcg tta tca gaa caa gaa ttg gtg gat tgt gat act    576
Gly Glu Leu Ile Ser Leu Ser Glu Gln Glu Leu Val Asp Cys Asp Thr
    45                  50                  55                  60

gga tat aac caa gga tgc aat gga gga ctt atg gac tat gca ttt gag    624
Gly Tyr Asn Gln Gly Cys Asn Gly Gly Leu Met Asp Tyr Ala Phe Glu
                    65                  70                  75

ttc ata atc aac aat ggc ggc att gat tct gat gag gat tac cca tac    672
Phe Ile Ile Asn Asn Gly Gly Ile Asp Ser Asp Glu Asp Tyr Pro Tyr
                80                  85                  90

cgt ggt gtt gat ggt aga tgc gac aca tat agg aaa aat gct aaa gtc    720
Arg Gly Val Asp Gly Arg Cys Asp Thr Tyr Arg Lys Asn Ala Lys Val
            95                  100                 105

gtt tct att gat gac tac gaa gat gtt cct gcc tat gat gag tta gcc 768
Val Ser Ile Asp Asp Tyr Glu Asp Val Pro Ala Tyr Asp Glu Leu Ala
110 115 120

ttg aaa aag gcc gtt gca aat cag ccc gtg agc gtt gct att gaa gga 816
Leu Lys Lys Ala Val Ala Asn Gln Pro Val Ser Val Ala Ile Glu Gly
125 130 135 140

ggg ggc agg gaa ttt caa tta tat gta tct ggt gta ttc acg ggg aga 864
Gly Gly Arg Glu Phe Gln Leu Tyr Val Ser Gly Val Phe Thr Gly Arg
145 150 155

tgt ggc aca gca cta gat cat ggt gtc gtg gct gtt ggg tat gga aca 912
Cys Gly Thr Ala Leu Asp His Gly Val Val Ala Val Gly Tyr Gly Thr
160 165 170

gct aaa ggt cat gat tat tgg atc gta agg aat tca tgg ggt tct agc 960
Ala Lys Gly His Asp Tyr Trp Ile Val Arg Asn Ser Trp Gly Ser Ser
175 180 185

tgg gga gag gat ggc tac atc aga tta gaa aga aat ctt gct aac agc 1008
Trp Gly Glu Asp Gly Tyr Ile Arg Leu Glu Arg Asn Leu Ala Asn Ser
190 195 200

aga tca ggc aag tgt gga att gca att gag cca tct tat ccc ctt aag 1056
Arg Ser Gly Lys Cys Gly Ile Ala Ile Glu Pro Ser Tyr Pro Leu Lys
205 210 215 220

aat ggt cca aat ccc cct aat cct gga cca tca ccc cct tca cct gtg 1104
Asn Gly Pro Asn Pro Pro Asn Pro Gly Pro Ser Pro Pro Ser Pro Val
225 230 235

aag ccg cca aat gtc tgt gac aac tac tac agc tgt gct gat agt gct 1152
Lys Pro Pro Asn Val Cys Asp Asn Tyr Tyr Ser Cys Ala Asp Ser Ala
240 245 250

act tgt tgc tgt att ttt gag ttc gga aat gct tgc ttc gag tgg ggt 1200
Thr Cys Cys Cys Ile Phe Glu Phe Gly Asn Ala Cys Phe Glu Trp Gly
255 260 265

tgc tgt cct ctt gag ggt gct tcc tgc tgt gat gac cac tac agt tgc 1248
Cys Cys Pro Leu Glu Gly Ala Ser Cys Cys Asp Asp His Tyr Ser Cys
270 275 280

tgc cct gca gac tat ccc atc tgc aac act tac gct gga act tgt ctc 1296
Cys Pro Ala Asp Tyr Pro Ile Cys Asn Thr Tyr Ala Gly Thr Cys Leu
285 290 295 300

agg agc aag aac aac ccc ttt gga gtg aag gca tta agg cgt act cca 1344
Arg Ser Lys Asn Asn Pro Phe Gly Val Lys Ala Leu Arg Arg Thr Pro
305 310 315

gcg aaa ccc cat tgg acc ttc gga cgt aag aac aag gtc agc agt gct 1392
Ala Lys Pro His Trp Thr Phe Gly Arg Lys Asn Lys Val Ser Ser Ala
320 325 330

<210> 7

<211> 464

<212> PRT

<213> Glycine max

<400> 7

Met Thr Met Ala Ala Ile Val Leu Leu Phe Thr Val Phe Ala Val Ser
-130 -125 -120

Ser Ala Leu Asp Met Ser Ile Ile Ser Tyr Asp Ser Ala His Ala Asp
-115 -110 -105

Lys Ala Ala Thr Leu Arg Thr Glu Glu Glu Leu Met Ser Met Tyr Glu
-100 -95 -90 -85

Gln Trp Leu Val Lys His Gly Lys Val Tyr Asn Ala Leu Gly Glu Lys
-80 -75 -70

Glu Lys Arg Phe Gln Ile Phe Lys Asp Asn Leu Arg Phe Ile Asp Asp
-65 -60 -55

His Asn Ser Ala Glu Asp Arg Thr Tyr Lys Leu Gly Leu Asn Arg Phe
-50 -45 -40

Ala Asp Leu Thr Asn Glu Glu Tyr Arg Ala Lys Tyr Leu Gly Thr Lys
-35 -30 -25

Ile Asp Pro Asn Arg Arg Leu Gly Lys Thr Pro Ser Asn Arg Tyr Ala
-20 -15 -10 -5

Pro Arg Val Gly Asp Lys Leu Pro Asp Ser Val Asp Trp Arg Lys Glu
-1 1 5 10

Gly Ala Val Pro Pro Val Lys Asp Gln Gly Gly Cys Gly Ser Cys Trp
15 20 25

Ala Phe Ser Ala Ile Gly Ala Val Glu Gly Ile Asn Lys Ile Val Thr
30 35 40

Gly Glu Leu Ile Ser Leu Ser Glu Gln Glu Leu Val Asp Cys Asp Thr
45 50 55 60

Gly Tyr Asn Gln Gly Cys Asn Gly Gly Leu Met Asp Tyr Ala Phe Glu
65 70 75

Phe Ile Ile Asn Asn Gly Gly Ile Asp Ser Asp Glu Asp Tyr Pro Tyr
80 85 90

Arg Gly Val Asp Gly Arg Cys Asp Thr Tyr Arg Lys Asn Ala Lys Val
95 100 105

Val Ser Ile Asp Asp Tyr Glu Asp Val Pro Ala Tyr Asp Glu Leu Ala
110 115 120

Leu Lys Lys Ala Val Ala Asn Gln Pro Val Ser Val Ala Ile Glu Gly
125 130 135 140

Gly Gly Arg Glu Phe Gln Leu Tyr Val Ser Gly Val Phe Thr Gly Arg

145 150 155

Cys Gly Thr Ala Leu Asp His Gly Val Val Ala Val Gly Tyr Gly Thr
160 165 170

Ala Lys Gly His Asp Tyr Trp Ile Val Arg Asn Ser Trp Gly Ser Ser
175 180 185

Trp Gly Glu Asp Gly Tyr Ile Arg Leu Glu Arg Asn Leu Ala Asn Ser
190 195 200

Arg Ser Gly Lys Cys Gly Ile Ala Ile Glu Pro Ser Tyr Pro Leu Lys
205 210 215 220

Asn Gly Pro Asn Pro Pro Asn Pro Gly Pro Ser Pro Pro Ser Pro Val
225 230 235

Lys Pro Pro Asn Val Cys Asp Asn Tyr Tyr Ser Cys Ala Asp Ser Ala
240 245 250

Thr Cys Cys Cys Ile Phe Glu Phe Gly Asn Ala Cys Phe Glu Trp Gly
255 260 265

Cys Cys Pro Leu Glu Gly Ala Ser Cys Cys Asp Asp His Tyr Ser Cys
270 275 280

Cys Pro Ala Asp Tyr Pro Ile Cys Asn Thr Tyr Ala Gly Thr Cys Leu
285 290 295 300

Arg Ser Lys Asn Asn Pro Phe Gly Val Lys Ala Leu Arg Arg Thr Pro
305 310 315

Ala Lys Pro His Trp Thr Phe Gly Arg Lys Asn Lys Val Ser Ser Ala
320 325 330

<210> 8
<211> 357
<212> DNA
<213> Artificial Sequence

<220>
<223> Description of Artificial Sequence:promoter region
of pUCTRPMTG(+)D2

<400> 8
cgcccaatac gcaaaccgcc tctccccgcg cgttggccgc ttcattaatg cagctggcac 60
gacaggtttc ccgactggaa agcgggcagt gagcgcaacg caattaatgt gagttagctc 120
actcattagg caccccaggc tttacacttt atgcttccgg atcgtatgtt gtgtggaatt 180
gtgagcggat aacaatttca cacaggaaac agctatgacc atgattacgc caagcttgca 240
tgcctgcagg tcgccctttc gtcttcaaga attcccctgt tgacaattaa tcatcgaact 300
agttaactag tacgcaagtt cacgtaaaaa gggtatcgat tagtaaggag gtttaaa 357

<210> 9
<211> 20
<212> DNA

<213> Artificial Sequence

<220>
<223> Description of Artificial Sequence:PCR primer for
      amplifying trp promoter region

<400> 9
cgcccaatac gcaaaccgcc                                                20

<210> 10
<211> 27
<212> DNA
<213> Artificial Sequence

<220>
<223> Description of Artificial Sequence:PCR primer for
      amplifying trp promoter region

<400> 10
tttaaacctc cttactaatc gataccc                                        27

<210> 11
<211> 32
<212> DNA
<213> Artificial Sequence

<220>

<223> Description of Artificial Sequence:PCR primer for
      amplifying D3 region

<400> 11
ggaggtttaa aatgtacgac agcgcccacg cg                                32

<210> 12
<211> 23
<212> DNA
<213> Artificial Sequence

<220>
<223> Description of Artificial Sequence:PCR primer for
      amplifying D3 region

<400> 12
caagcttgta ggttcggtcc tcc                                          23

<210> 13
<211> 25
<212> DNA
<213> Artificial Sequence

<220>
<223> Description of Artificial Sequence:PCR primer for
      linking trp promoter region to D3 region

<400> 13
tacgacagcg cccacgcgga caagg                                                25

<210> 14
<211> 37
<212> DNA
<213> Artificial Sequence

<220>
<223> Description of Artificial Sequence:PCR primer for
      linking trp promoter region to D3 region

<400> 14
gggatcctca gctgtagtag ttgtcacaga catttgg                                   37

## Description

### Background of the Invention

The present invention relates to an angiotensin converting enzyme inhibitor, particularly an inhibitor peptide for angiotensin converting enzyme and a method of producing the inhibitor. More specifically, the present invention relates to an inhibitory peptide for angiotensin converting enzyme which is capable of being orally ingested and a method of producing the peptide. The present invention further relates to an antihypertensive containing an angiotensin converting enzyme inhibitor, particularly an inhibitory peptide for angiotensin converting enzyme.

Angiotensin converting enzyme generates angiotensin II having vasopressor activity including vasoconstriction by cleaving C-terminal His-Leu of inactive angiotensin I. It is a vasopressor enzyme also having the activity of degrading bradykinin which has strong vasodilator action. It is known that hypertension can be treated by inhibiting the function of angiotensin converting enzyme. An angiotensin converting enzyme inhibitor was originally found in a snake poison as a peptide which enhances ileum smooth muscle contraction in guinea pig caused by bradykinin and was called as bradykinin potentiator.

In the later studies, it was elucidated that a fraction having angiotensin converting enzyme inhibitor activity existed in the degradation product of food proteins such as pepsin hydrolysates of soybean proteins or ginseng proteins. As such an example, for example, a peptide having hypotensive function was found among the peptide fragments in human β casein. Furthermore, angiotensin converting enzyme inhibitory peptides are also found among the hydrolysates which had been produced with proteolytic enzymes. Such peptides together with their sequences are reported in, for example, Publication of Unexamined Japanese Patent Application (JP-Kokai) No. 8-225593 (pepsin degradation products of soybean proteins), JP-Kokai No. 8-269088 (pepsin degradation products of κ casein glycomacropeptide), JP-Kokai No. 11-29594 (thermoase PC-10 degradation product of tuna fish meat), JP-Kokai No. 11-335393 (pepsin degradation products of ginseng) and JP-Kokai No. 2000-4799 (endo-proteinase and exo-proteinase degradation products of potato protein). The angiotensin converting enzyme inhibitory peptides which have been reported thus far generally tend to contain Pro residues and are frequently the peptides having high hydrophobicity.

On the other hand, independent of these studies, various proteolytic enzymes are used to modify the properties of proteins for food products. For example, the applicant reported a thiol protease having high proteolytic activity toward proteins, particularly proteins for food products (JP-Kokai No. 8-264) and the applicant also reported that the hydrolysates having low bitterness were prepared with the protease (protease D3) on proteins (JP-Kokai NO. 12-83695).

### Summary of the Invention

It has been literary reported that the substances having angiotensin converting enzyme inhibitory activity, particularly the peptides having angiotensin converting enzyme inhibitory activity, existed in the degradation products of proteins for foods, as described above. However, the enzymatic hydrolysates disclosed in these references are produced with protease formulations such as pepsin or papain, and are known as having strong bitterness. Thus, orally ingesting these hydrolysates as the product containing angiotensin converting enzyme inhibitor peptides will be limited in the light of their taste.

Accordingly, the object of the present invention is to provide a peptide having an angiotensin converting enzyme inhibitory activity with improved in the taste, and to provide a method of producing an angiotensin converting enzyme inhibitors comprising at least one of such peptides.

Another object of the present invention is to provide an antihypertensive comprising the peptide having angiotensin converting enzyme inhibitory activity.

The inventors of the present invention have found that the protease D3 derived from germinating soybean cotyledons is an enzyme which can produce hydrolysates having less bitterness than those produced with other commercially available enzymes (JP-Kokai No. 2000-83695) and that angiotensin converting enzyme inhibitor existed in the soy protein hydrolysates with D3. Further investigation of the hydrolysates led to the establishment of the present invention.

Accordingly, the peptide of the present invention which has an angiotensin converting enzyme inhibitory activity and which is improved in the taste is any of the five peptides represented by the following formula (1) to (5) and the salts thereof:
(1) Tyr-Val-Val-Phe-Lys (SEQ ID NO:1),
(2) Pro-Asn-Asn-Lys-Pro-Phe-Gln (SEQ ID NO:2),
(3) Asn-Trp-Gly-Pro-Leu-Val (SEQ ID NO:3),
(4) lle-Pro-Pro-Gly-Val-Pro-Tyr-Trp-Thr (SEQ ID NO:4),
(5) Thr-Pro-Arg-Val-Phe (SEQ ID NO:5),
wherein Tyr represents tyrosine, Val represents valine, Phe represents phenylalanine, Lys represents lysine, Pro represents Proline, Asn represents asparagine, Gln represents glutamine, Trp represents tryptophan, Gly represents Glycine, Leu represents leucine, lie represents isoleucine, Thr represents threonine, Arg represents arginine, respectively.

The method according to the present invention is the method of producing hydrolysates characterized in that a protein, particularly a soybean protein, is reacted with protease D3.

The present invention is also an antihypertensive comprising at least one of these peptides or the salts thereof.

### Brief Description of the Drawings

Fig. 1 . is the schematic diagram showing the procedures of constructing plasmid pUCTRPproD3-N. pUCTRPproD3-N contains the promoter region derived from pUCTRPMTG(+)D2trp and the sequence encoding the pro-part of D3.

Fig. 2 is the schematic diagram showing the procedures of constructing plasmid pUC-D3-C. pUC-D3-C contains the sequence encoding the sequence of the pro-part and mature D3 sequence.

Fig. 3 is the schematic diagram showing the procedures of constructing plasmid pUCTRPproD3. pUCTRPproD3 contains the promoter region derived from pUCTRPMTG(+)D2trp, the sequence encoding the pro-sequence of D3 and mature D3 sequence.

Fig. 4 shows the chromatography on gel filtration and angiotensin converting-enzyme inhibitory activity of soybean protein hydrolysates with protease D3. Open square (□) represents the absorbance at 215nm and black circle (●) represents the inhibition rate (%) for the enzyme.

Fig. 5 shows the chromatography on reverse-phase column chromatography and angiotensin converting enzyme inhibitory activity of the fractions that exhibited angiotensin converting enzyme inhibitory activity after gel-filtration of soybean protein hydrolysates solution with protease D3. Open square (□) represents the absorbance at 215nm and black circle (●) represents the inhibition rate (%) for the enzyme.

Fig. 6 shows the hypotensive effect of the soybean protein hydrolysates solution prepared with protease D3. Black diamond represents the change in the blood pressure in SHR rats for the case in which the control solution was given, black rectangular (■), black triangle (▲), cross mark (X) and black circle (●) represent the case in which 100mg/kg, 500mg/kg and 1000mg/kg of soybean protein hydrolysates prepared with D3 was given, respectively.

### Description of the Preferred Embodiments

The hydrolysates containing the peptides of the present invention may be easily prepared in large quantity with protease D3.

Protease D3 used for producing hydrolysates containing the peptides of the present invention is the proteolytic enzyme which allows the production of peptides having low bitterness, as described above. Protease D3 may be obtained from, for example, germinating soybean cotyledons at about day 10 of germination according to the method described in JP-Kokai No. 8-264. Alternately, recombinant protease D3 (rD3) can be also obtained by autocatalytic processing of recombinant inactive protease proD3 having pro-sequence. Recombinant proD3 may be produce in, for example, E.coli, yeast or Aspergillus. Protease D3 derived from germinating soybean cotyledons is described in JP-Kokai No. 9-121870. In JP- Kokai No. 9-121870, D3-α and D3-β are described as recombinant D3s, both of which may be used for producing the peptides of the present invention.

More specifically, rD3 may be produced by using trp promoter, as the promoter which can be easily induced by Tryptophan defective medium and the clone pUCa (JP-Kokai No. 9-12-121870, FERM NP-7835 (originally deposited as FERM P-14687) (Independent Administrative Agency, National Institute of Advance Industrial Science and Technology, Tsukuba Central 6, 1-1, Higashi 1-Chome Tsukuba-shi, Ibaraki-ken, 305-8566 Japan)). Optionally, it is possible to modify the sequence of promoter region or of the further upstream region or to change the DNA sequence of D3 gene taking the codon usage into account.

Trp promoter is a strong promoter. For example, plasmid pTTG2-22 (JP-Kokai No.6-225775), which highly expressed *Crysophrys major* transglutaminase (TG) gene, used trp promoter where the upstream region of *Crysophrys major* TG was designed for high level expression of heterologous proteins in E.coli. Additionally, plasmid pUCTRPMTG(+)D2 (JP-Kokai No. 11-75876), which highly expressed microbial transglutaminase (MTG) gene, used the upstream region containing trp promoter of this *Crysophrys major* TG expression plasmid, and the region was introduced into multi-copy plasmid pUC19 to highly express MTG. For the purpose of producing proD3 in the present invention, the promoter regions of these plasmids are suitable.

The conversion into high-active D3 by autocatalytic activation may be conducted in the condition described in JP-Kokai No. 9-121870, namely by subjecting proD3 to 200mM NaCl solution at about pH 4, and incubating at between about 30°C and about 50°C, preferably at between about 35°C and about 45°C. This enzyme may be reacted with proteins at pH 3 to pH 7, preferably at pH 3.5 to 5.5, at about 30°C to 50°C, preferably at about 35°C to 45°C. Protease D3 can hydrolyze proteins very efficiently at these conditions to generate the peptides of the present invention.

The peptides of the present invention may be produced with D3 on soybean proteins, particularly soybean storage proteins. The substrates for protease D3 may be purified soybean proteins, but the products obtained by merely grinding or suspending proteins, optionally processing proteins with acids or alkali or those followed by removing the insoluble fractions by centrifugation or filtration can also be used. The degradation may be conducted by adjusting the substrate solution such as those described above to about pH 3 - 7, preferably about pH 3.5 - 5.5, particularly preferably about pH 4.0 - pH 5.0, adding to the solution rD3 prepared as described above such that the ratio of substrate to enzyme (substrate/enzyme) will be about 100/1 - 1000/1 and incubating for 6 - 60 hours at about 30°C to about 50°C, preferably at about 35°C to about 45°C. After incubating, the supernatant can be obtained by, for example, centrifugation after inactivating the enzyme by, for example, heating. The supernatant may be neutralized with, for example, NaOH, and recovering the hydrolysates by, for example, freeze-drying, to obtain crude hydrolysates containing the peptides of the present invention. The crude proteolytic degradation product containing the peptides of the present invention may be further purified. For purification, the fact that the peptides of the present invention have the molecular weight raging 654 to 1029 may be utilized. For example, the high molecular weight fraction having the molecular weight of 2000 or more may be removed by gel filtration chromatography and the low molecular weight fraction may be further fractionated by reverse-phase chromatography. The angiotensin converting enzyme inhibitory activity in these fractions may be confirmed as follows. Briefly, substances which may be the substrate of angiotensin converting enzyme such as p-hydroxyl benzoyl glycyl-L-histidyl -L-leucine, an angiotensin converting enzyme such as the commercially available rabbit pulmonal angiotensin converting enzyme and the aforementioned partially purified fraction to give rise to the reaction and determined the activity by colorimetric analysis of the reaction solution.

In one embodiment of the present invention, the peptides of the present invention are obtained by further purifying the hydrolysates obtained by the methods such as the aforementioned method or by further purifying the partially purified fractions of the hydrolysates. The techniques for purifying proteins are well known, which include ion-exchange chromatography, reverse phase high-performance liquid chromatography, affinity chromatography and the like.

In another embodiment of the present invention, the peptides of the present invention are chemically synthesized. The peptides of the present invention can be synthesized according to the conventional peptide synthesizing methods such as solid phase synthesis. The thus synthesized peptides may be purified by, for example, ion-exchange chromatography, reverse phase high-performance liquid chromatography, affinity chromatography and the like. Such solid phase synthesis techniques and the subsequent purification of peptides are well known in the art.

Thus prepared angiotensin converting enzyme inhibitor peptides may be used in any type of food products, beverages or medical diet. Additionally, the hydrolysates prepared according to the methods of the present invention may be used as the materials for foods after being appropriately processed dependent on the purpose. The angiotensin converting enzyme inhibitory peptides of the present invention may be used as antihypertensive. The peptides or the hydrolysates of the present invention, alone or together with various pharmaceutically acceptable carriers or additives, may be orally or parenterally administered, but it is preferable to administer them orally, and most preferably they will be administered by mixing them into foods or beverages as described above. The suitable dosage for aforementioned purpose may be determined depending on the intended therapeutic effects, the manner of administration, the duration of therapy, the age and the body weight. For oral administration, 100mg/(kg body weight) to 1000mg/(kg body weight) or more is preferable as one dosage. The angiotensin converting enzyme inhibitory peptides of the present invention has no adverse effects or has minimum adverse effects, if any, and they can be therefore ingested more than 1000mg/kg body weight or ingested plural times a day with foods or beverages over a long period. The other methods for oral administration or the formulations for parenteral administration may be obtained according to the conventional formulation methods depending on the purpose and the manner of administration by mixing with, for example, inert diluents such as lactose, calcium carbonate or calcium phosphate, swelling agents such as alginic acid, cornstarch or starch, sweetening agents such as sucrose, lactose or saccharine, aromatics, coloring agents, humectants such as magnesium stearate and talc or excipients such as wax.

### Examples

### Reference example 1. Generation of the genetic construct for expressing proD3

A part of D3-β cDNA (SEQ ID NO:6), which had been cloned from cDNA library prepared with mRNA obtained from germinating soybean cotyledons, was introduced into an expression vector being capable of functioning in E.coli.

### (1) Ligation of trp promoter and D3 gene

For D3 gene, the clone pUCa (JP-Kokai No. 9-121870, FERM BP-7835 (originally deposited as P-14687)) was used wherein the full length D3-β cDNA had been integrated.

The integration of DNA fragment was conducted by PCR to ligating trp promoter to the upstream region of D3-β cDNA. Firstly, the region containing trp promoter (SEQ ID NO:8) from MTG expression plasmid pUCTRPMTG(+)D2 (JP-Kokai No. 11-75876) and the partial region of the D3 cDNA from pUCa were amplified by PCR. The primers for amplifying trp promoter were TRP-N2 (SEQ ID NO:9) and TRP-C2 (SEQ ID NO:10) and the primers for partially amplifying D3 were D3-N (SEQ ID NO: 11) and D3-C (SEQ ID NO:12)3 wherein TRP-N2 and D3-N were sense primers, and TRP-C2 and D3-C were antisense primers. D3-N was designed such that it could connect 11 bases sequence for ligation with the sequence containing trp promoter and the initiating ATG codon to the sequence starting at TAC encoding Tyr at position -107 (supposed to be the starting point of pro-sequence) of D3 indicated as SEQ ID NO:6. This sequence is complementary to the sequence in TRP-C2. Hindlll site was introduced into D3-C.

Firstly, PCR was conducted for pUCTRPMTG(+)D2 using the primer TRP-N2 and TRP-C2, and for pUCa using the primer D3-N and D3-C, under the condition of 35 cycles of [94°C for 30 seconds, 72°C for 2 minutes], respectively. Each PCR product was extracted with phenol/chloroform, precipitated with ethanol and was dissolved in 100 µl of H₂O.

1µl from each PCR product was taken and mixed together, heat denatured at 94°C, and was subjected to PCR under the condition of 35 cycles of [94°C for 30 seconds, 55°C for 1 minute and 72°C for 2 minutes] using the primer TRP-N2 and D3-C.

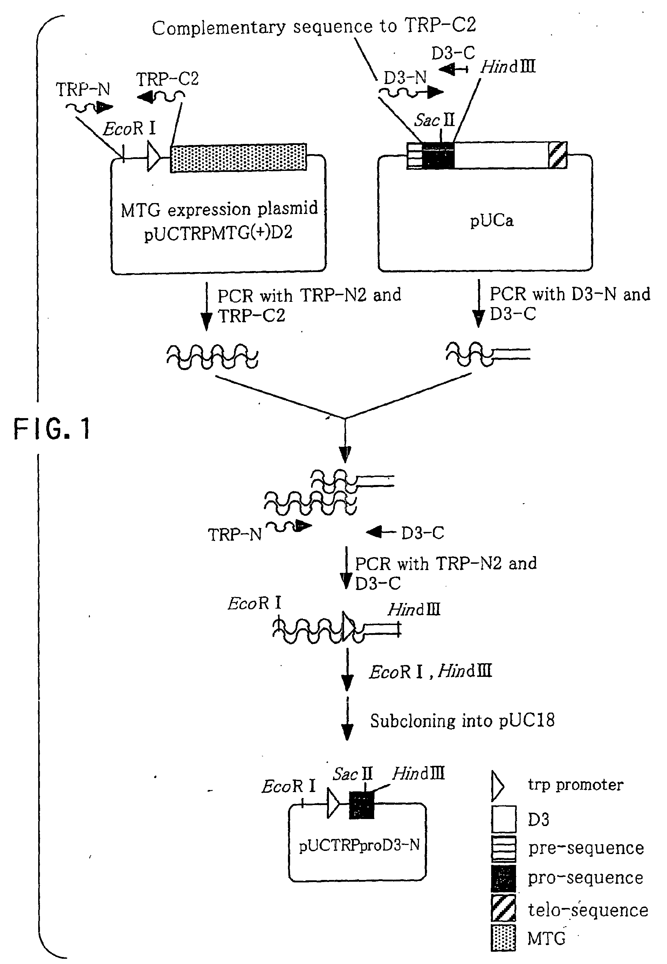

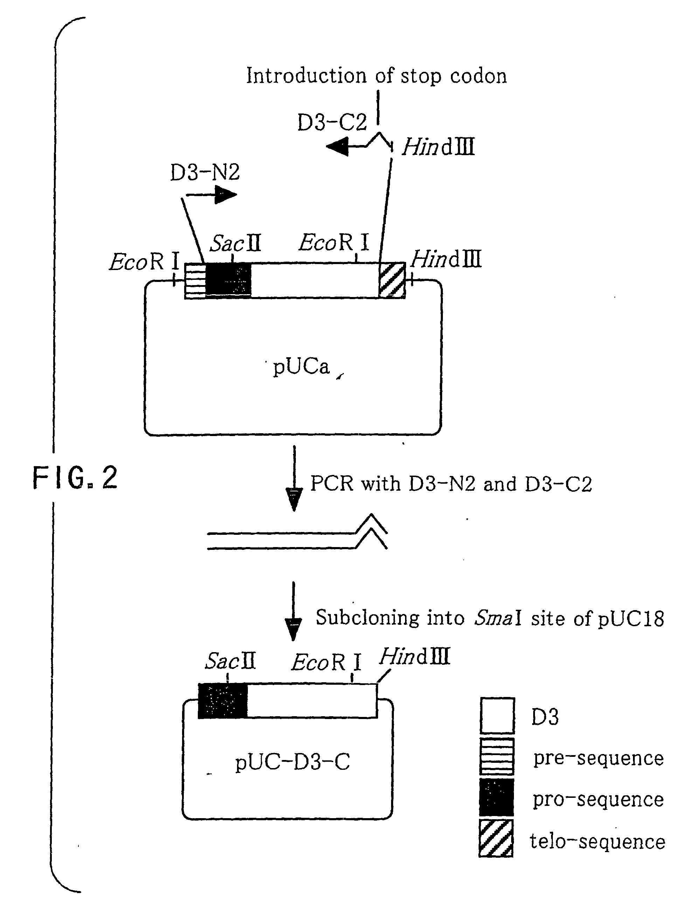

The product of the second PCR was extracted with phenol/chloroform, precipitated with ethanol, digested with Hindlll and EcoRl, and subcloned into pUC 18 to obtain pUCTRPproD3-N (Fig. 1), the sequence of which was confirmed.

### (2) Introduction of stop codon into the C-terminal sequence

PCR was conducted to introduce the stop codon at the C-terminal of D3-β. The sense primer D3-N2 (SEQ ID NO:13) and the antisense primer D3-C2 (SEQ ID NO:14) were used. D3-C2 is the primer for mutating TGT encoding Cys of residue No. 248 in the amino acid sequence indicated as SEQ ID NO:6 to TGA, the stop codon.

PCR was conducted for pUCa using the primers D3-N2 and D3-C2 under the condition of 35 cycles of [94°C for 30 seconds, 55°C for 1 minute and 72°C for 3 minutes]. Each PCR product was extracted with phenol/chloroform and was cloned into Smal site of pUC18 to obtain pUC-D3-C (Fig. 2), of which sequence was confirmed.

### <Sequence listing free text>

SEQ ID NO:8: promoter region of pUCTRPMTG(+)D2
SEQ ID NO:9 and SEQ ID NO:10: PCR primer for amplifying trp promoter region
SEQ ID NO: 11 and SEQ ID NO: 12: PCR primer for amplifying D3 region
SEQ ID NO:13 and SEQ ID NO:14 PCR primer for ligating trp promoter region to D3 region

### (3) Construction of proD3 expression plasmid

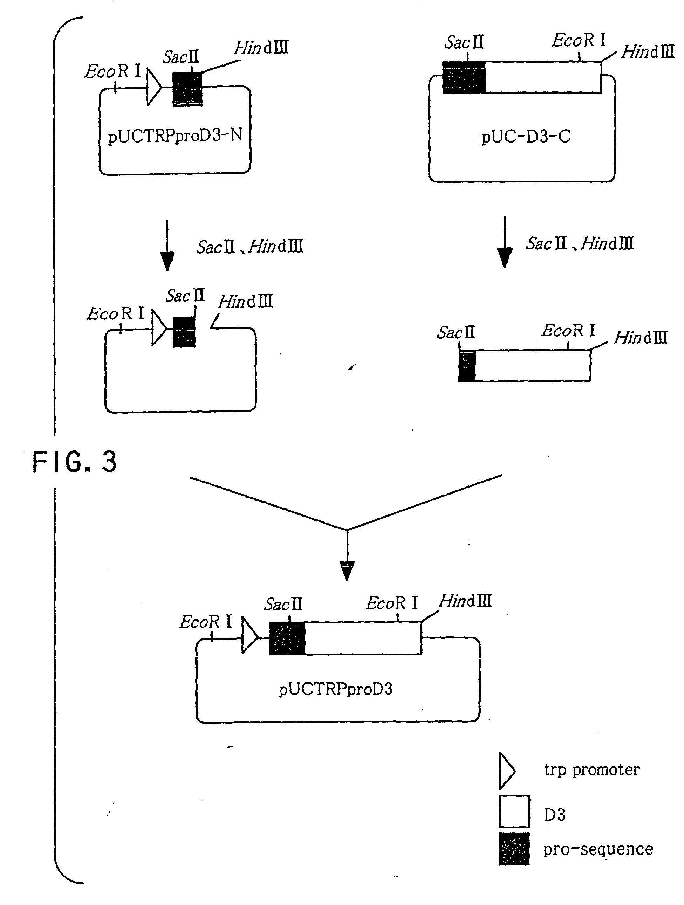

ProD3 expression plasmid driven by trp promoter was generated by using the partial fragment of thus obtained plasmid. Namely, Sacll-Hindlll fragment (large) of pUCTRPproD3-N and SacII-HindIII fragment (small) of pUC-D3-C were ligated to obtain pUCTRPproD3 (Fig. 3).

### Reference Example 2. Expression of proD3 and production of mature D3

The proD3 expression plasmid obtained according to Reference Example 1 was expressed in E.coli to finally obtain D3 in its active form.
(1) E.coli strain JM109 (Takarashuzo CO., Ltd.) was transformed with pUCTRPproD3 and was selected on agar plates containing ampicillin to obtain transformants. The transformants were inoculated on M9-casamino acid medium containing ampicillin and cultured at 37°C for about 20 hours. Consequently, the intended D3 gene product was accumulated as protein inclusion bodies within the cells.

After harvesting, the cells were sonicated and the protein inclusion bodies were recovered by centrifugation. The protein inclusion bodies were washed and dissolved in 8M urea - 10mM dithiothreitol - 50mM NaCI - 50mM Tris-HCI - 5mM disodium ethylenediaminetetraacetate solution (pH 8). The resulted solution was also referred to as "solubilized proD3 solution". The three-dimensional structural reconversion (refolding) of the denatured proD3 in the solubilized proD3 solution into proD3 having the naturally occurring three-dimensional structure was carried out according to the following procedures.

2.5ml of solubilized proD3 solution was added to PD-10 column (Amersham Bioscience), which had been previously equilibrated with 1mM reduced glutathione - 0.1 to 0.5mM oxidized glutathione - 50mM potassium phosphate - 5mM disodium ethylenediaminetetraacetate solution (pH 10.5) and then 3.5ml of 50mM potassium phosphate (pH 10.5) was added. The eluent was recovered as refolded proD3.

### (2) Autocatalytic activation

The solution of refolded proD3 was incubated at 37°C and at pH of about 4.5. As a result, proD3 having the molecular weight of about 41 k was converted to highly active D3 having the molecular weight of about 30k. This highly active D3 was used for proteolytic hydrolysis.

### Example 1 Preparation of D3-degradation products

The isolated soybean protein solution was adjusted to about pH 4.5 with hydrochloric acid. D3 was added to the isolated soybean protein solution such that the ratio substrate/enzyme = 500/1 and allow the reaction at 37°C for 24 hours. After the reaction was completed, the enzyme was inactivated by heating and the supernatant was obtained by centrifugation. The pH of the supernatant was neutralized to around the neutral point with NaOH and the hydrolysates were obtained through freeze-drying.

### Example 2. Identification of the candidates of angiotensin converting enzyme inhibitory peptides

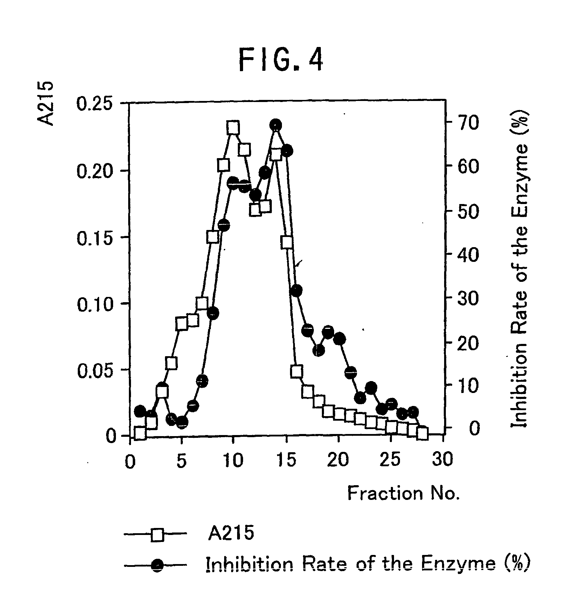

The peptides prepared as described in Example 1 were dissolved to the concentration of 5mg/ml, subjected to gel filtration column (Superdex75 HR 10/30; Amersham Bioscience) after filtration, and the fractions having the molecular weight of 2000 or less were divided to seven fractions. 0.05% TFA was used as a mobile phase, and the flow rate was 0.5ml/min. and the detection was carried out by determining the absorbance at 215nm. The angiotensin converting enzyme inhibitory activity of each fraction was determined and five fractions, G1 to G5 (Fig. 4; G1 fraction: fractions No.10 to No.11; G2 fraction: fractions No.12 and 13; G3 fraction: fractions No.14 and 15; G4 fraction: fractions No. 16 and 17; G5 fraction: fractions No. 18-21), where the inhibitory activity was observed, were dried using centrifugal evaporator.

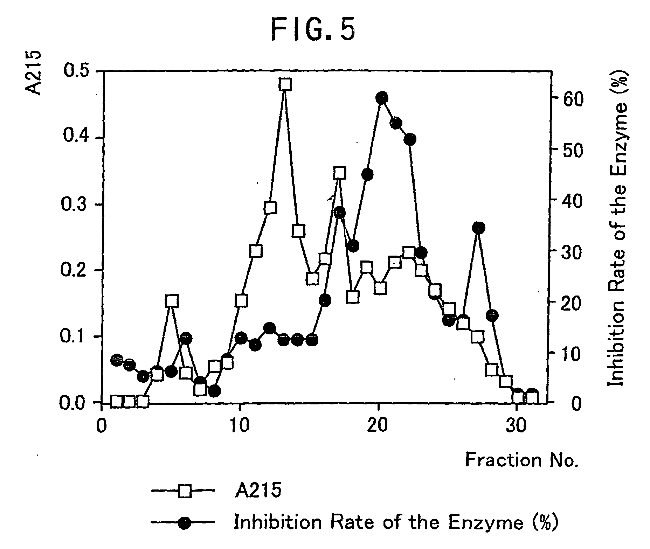

The active fractions obtained by gel filtration were then fractionated by using reverse column COSMOSIL 5C18 ARA4.6/250 (Nakarai Tesque). Solution A (distilled water containing 0.05% TFA) and solution B (acetonitrile containing 0.065% TFA) was used as the mobile phase, and the chromatography was carried out using concentration gradient technique where the concentration of solution B increased from 0% to 50% for 50 minutes with the flow rate of 0.75ml/min. 0.75ml aliquots were collected. The results showed that the angiotensin conversion enzyme inhibitory activity tend to be high when the concentration gradient of solution B is in the range from about 20% to 30% of solution B (Fig. 5).

These fractions were subjected to MS/MS analysis to determine the sequences of the peptides, which resulted in obtaining 74 candidates for angiotensin converting enzyme inhibitory peptides.

Among the candidate peptides, five peptides were selected which were expected from the sequence to be derived from storage proteins taking large scale preparation into account, and which contained Pro residue or which were expected to have high hydrophobicity.

### Example 3. Synthesis of the candidate peptides as angiotensin converting enzyme inhibitor

To elucidate whether or not these candidate peptides have angiotensin converting enzyme inhibitory activity, the peptides were synthesized by solid phase method. The procedure of synthesizing Tyr-Val-Val-Phe-Lys is described below. 50mg of Fmoc-Lys(Boc) resin (p-alkoxy benzyl alcohol resin into which Fmoc-Lys(Boc)-OH was introduced at a ratio of 0.48mmol/g resin; Nova Biochem) was suspended in DMF (1 ml) and the suspension was shaken for 1 hour to allow the resin to swell.

The resin was subjected to the following Fmoc group removing cycle and Fmoc amino acid condensation cycle.
a) shaking in 1ml of DMF for 1 minute (once);
b) shaking in 600 µl of 50% piperidine-DMF solution for 12 minutes:
c) washing 5 times with 600µl of DMF;
d) wash once with 1 ml of isopropanol;
e) allowing the resin obtained at Fmoc group removing cycle to swell by shaking in 1ml of DMF twice;
f) Fmoc-Phe-OH (15mg) and HOBt (8mg) were dissolved in 800µl and added to the resin, 50 µl of 1M dicyclohexylcarbadiimide methylene chloride solution was then added and was shaken for 60 minutes;
g) washing twice with 1ml of DMF; and,
h) washing twice with isopropanol.

Fmoc group removing cycle (a - d) and Fmoc amino acid condensation cycle (f - h) were repeated similarly to condense Fmoc-Val-OH, Fmoc-Val-OH and Fmoc-Tyr(But)-OH (all of them were supplied by Nova Biochem) sequentially to obtain Fmoc-Tyr(But)-Val-Val-Phe-Lys(Boc)resin.

Fmoc group was then removed by treating the protected peptidyl resin with 50% piperidine-DMF solution and the product was subjected to the following deprotection step:
a) shaking for 12 minutes after adding 1ml of ether;
b) drying by using centrifugal evaporator;
c) shaking for 60 minutes after adding 1ml of phenol-water-thioanisole-etahnediol-TFA (1.42 : 1.4 : 1.5: 0.9: 24.9);
d) filtrating the resin, washing twice the residual resin with 1ml of TFA and combining the filtrate;
e) adding 10ml of ether and 1ml of water to fractionate aqueous phase;
f) further washing the aqueous phase twice; and,
g) freeze drying to obtain crude peptide.

The obtained crude peptide was purified to exhibit a single peak in reverse phase chromatography analysis using Inertsil ODS column (GL Science). Additionally, the results of mass spectrometry analysis consisted with the theoretical values. The similar reaction and process as described above were carried out for other peptides to synthesize the peptides shown in Table 1.

Table 1. Angiotensin converting enzyme inhibitor peptide candidates
Peptide | Mass spectroscopy | (Theoretical value)
Tyr-Val-Val-Phe-Lys | 655.2 | (654.8)
Pro-Asn-Asn-Lys-Pro-Phe-Gln | 844.6 | (843.9)
Asn-Trp-Gly-Pro-Leu-Val | 685.6 | (684.8)
Ile-Pro-Pro-Gly-Val-Pro-Tyr-Trp-Thr | 522.5 | (521.6)
Thr-Pro-Arg-Val-Phe | 655.6 | (654.8)

### Example 4. Determination of the inhibitory activity of Angiotensin converting enzyme inhibitory peptide candidates

To 50µl of each peptide solution sample, 125µl of 10mM p-hydroxyl benzoyl glycyl-L-histidyl-L-leucine, 2.5mM 4-aminoantipyrine, 3 units/ml hipricase (solution in 0.2M borate buffer containing 0.7M NaCI) were added and pre-incubated at 37°C for 3 minutes. 20 µl of 200mU/ml rabbit pulmonal angiotensin converting enzyme (Sigma) was added to the solution to start the reaction. After incubating at 37°C for 20 minutes, the reaction was stopped by adding 375 µl of 3mM EDTA, 0.2% Triton X-100, 6.5mM of sodium periodate solution. After incubating for further 3 minutes for color development. The reaction solution was subjected to colorimetry at a wavelength of 505nm. Distilled water was used as a control. The concentration exhibiting 50% inhibition was indicated in Table 2 as IC₅₀ values. The IC₅₀ of Bradykinin potentiator C (Peptide Institution) as determined by this method was shown as the positive control.

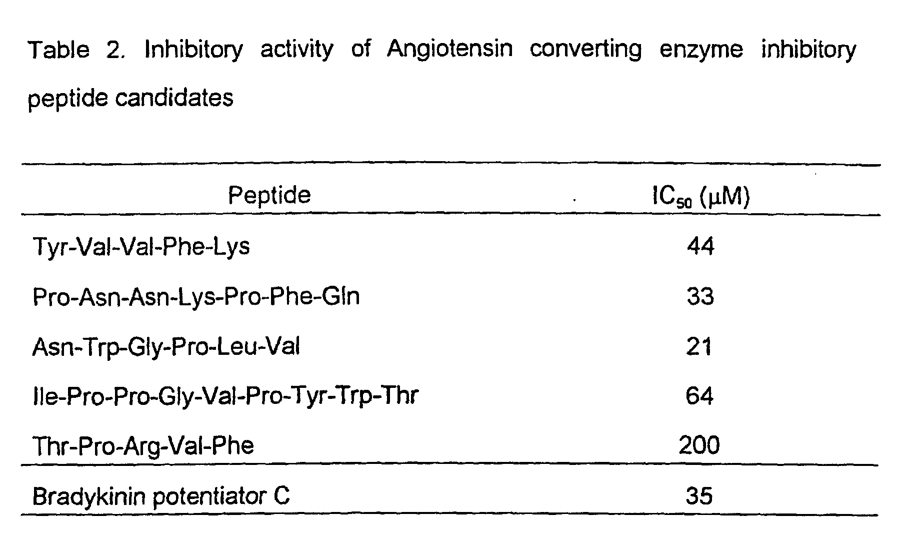

### Example 5. Determination of hypotensive activity of D3-degradation product

### (1) Preparation for hydrolysates with D3

Isolated soybean protein (AP-SU, Ajinomoto Co., Inc.) were dissolved in 50ml of deionized water, denatured at 120°C for 20 minutes and adjusted to pH 4.5. D3 was added to 0.5% of the substrate and allowed to react on the substrate at 40°C for 48 hours. After the reaction was completed the mixture was centrifuged and the pH of the supernatant was adjusted to about neutral point. The supernatant was then heated to 100°C for 10 minutes to inactivate the enzyme. After cooling on ice, desalination was carried out by using an electrodialyzer (Micro Acilyzer G3, Asahikasei, AC-110-800 cartridge); the product was freeze-dried and stored. The average molecular weight of the obtained hydrolysates was 1053. IC₅₀ value, the concentration for 50% inhibition against ACE, was 180µg/ml as measured according to the same method as described in Example 4.

### (2) Determination of hypotensive effect of hydrolysates

The 9 weeks old spontaneously hypertensive model rats (SHR/lzm:SPF) were purchase from Japan SLC Co. The rats were pre-fed including quarantine of one week. The animals which did not exhibit any abnormality were used to estimate the hypotensive effect of hydrolysates. The animals were kept separately in movable stainless rack in the environment of 22±3°C, 50±20% humidity, 12 hours light/dark cycle (8:00 - 20:00) and 13-17 times/hour ventilation. The animals were fed with solid diet, Labo MR Stock (Nihon Nousan Industry, Co.), by stainless solid diet feeder and, for the water, the animals received tap water through a polysulphone waterer, and both of them were given ad libitum. The amount of the soybean protein hydrolysates in each group was shown in Table 3. The hydrolysates were dissolved in distilled water (Otuka Distilled Water, Otuka Pharmaceutical Co., Ltd.). The control group received only the distilled water. 4 hours after receiving them, the rats were given 9g of feed, when the measurement was finished.

**Table 3.**

| The amount of hydrolysates given in each group | | | |
|---|---|---|---|
| Group | Received amount (mg/kg) | Received Volume (ml/kg) | Number of animals |
| Control | 0 | 10 | 7 |
| soybean protein hydrolysates | 50 | 10 | 7 |
| | 100 | 10 | 7 |
| | 500 | 10 | 7 |
| | 1000 | 10 | 7 |

The tests were carried out by determining the blood pressure and heart rate of 10 weeks old SHR rat by using noninvasive automated blood pressure manometer for small animals (MK-2000, Muromachi Kikai, Co.). Classification was carried out by stratified sequential randomized controlled trial using systolic blood pressure as the indicator. After grouping, the rats were starved overnight, and then the test samples were orally injected at 10ml/(kg body weight). After 0, 1, 2, 4 and 6 hours, the blood pressure and heart rate of the rats were determined. The significant difference between each group was determined by comparing the average values for each group which received the sample against the control group using Tukey-Kramer method (StatView-J 5.0) and the significant difference was to be recognized when the significance level was 5% or less.

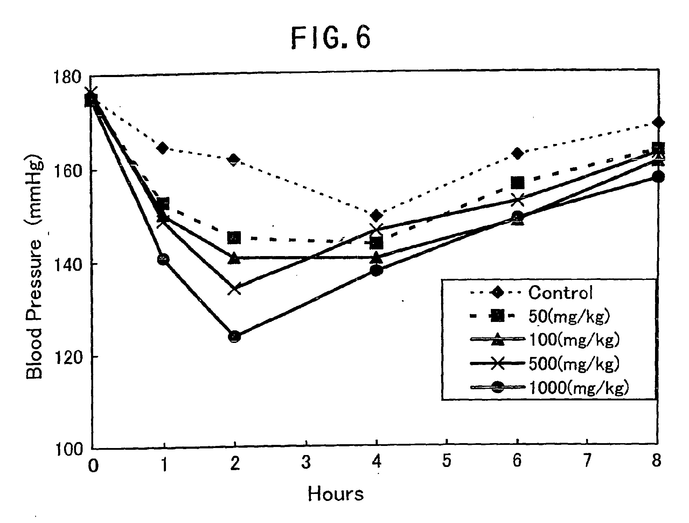

When SHRs received 50, 100, 500 and 1000mg/(kg body weight) of soybean protein hydrolysates, they exhibited the blood pressure decrease starting at 1 hour after giving, when compared with the control group. Regarding dose response, the statistical significant difference was not observed between 50mg/(kg body weight) group and the control group. The group received 100mg/(kg body weight) exhibited the significant decrease compared with the control group only at 1 hour after the administration, and the group received 500mg/(kg body weight) and 1000mg/(kg body weight) exhibited the significant reduce compared with the control group at 1 and 2 hours after administration. The results were shown in Table 4 and Figure 6.

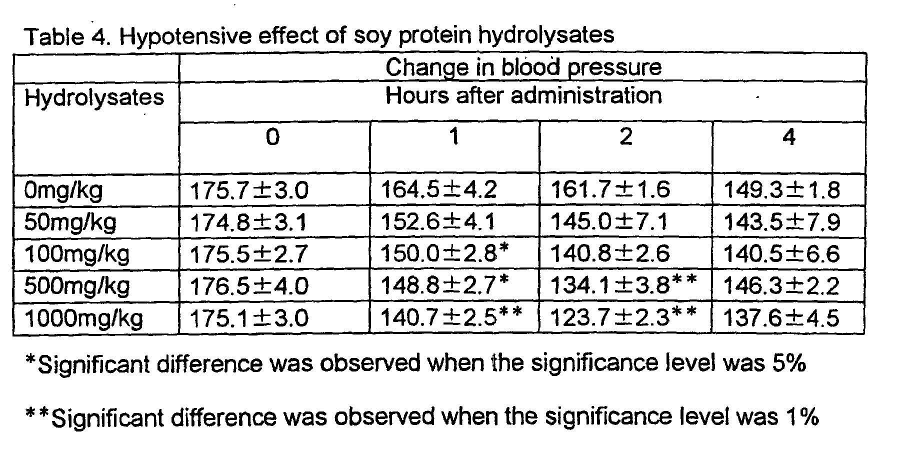

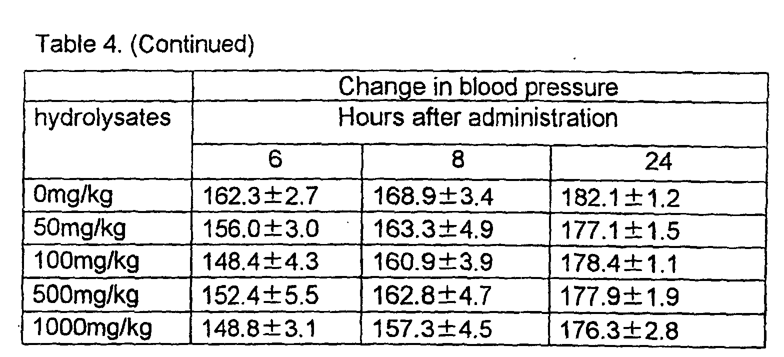

The changes in heart rate in these groups were not observed, although the data were not shown.

From these data, soybean protein hydrolysate was shown to exhibit significant hypotensive effect in vivo at the administration of 100mg/(kg body weight) or more in SHR and the dosage response was observed.

According to the present invention, biologically active peptides, namely, angiotensin converting enzyme inhibitor peptides, which are derived from soybean protein and which have improved taste, are provided. Additionally, according to the method of present invention for producing angiotensin converting enzyme inhibitor peptides, enzymatic hydrolysates containing angiotensin converting enzyme inhibitor peptides having low bitterness can be produced. These soybean protein hydrolysates containing these peptides have the hypotensive effect. Thus, the angiotensin converting enzyme inhibitory peptides of the present invention and the hydrolysates containing the peptides can be used for various foods including healthy foods having hypotensive effect.

## Claims

1. A peptide represented by any of the following formula (1 ) to (5) or the salts thereof:
(1)Tyr-Val-Val-Phe-Lys;
(2)Pro-Asn-Asn-Lys-Pro-Phe-Gln;
(3)Asn-Trp-Gly-Pro-Leu-Val;
(4)lle-Pro-Pro-Gly-Val-Pro-Tyr-Trp-Thr;
(5)Thr-Pro-Arg-Val-Phe.

2. An angiotensin inhibitor comprising the peptides according to claim 1 or the salts thereof.

3. The angiotensin inhibitor **characterized in that** it is a hydrolysate of soybean proteins.

4. A method of producing the angiotensin inhibitor which comprises hydrolyzing a soybean protein with D3.

5. An antihypertensive comprising the peptide of claim 1 or the salts thereof.

6. A food comprising the peptides according to claim 1 or the salts thereof.
